# EUROPEAN PATENT SPECIFICATION

(11) **EP 3 059 588 B1**
(45) Date of publication and mention of the grant of the patent: **10.01.2018**
(21) Application number: 15382066.7
(22) Date of filing: 18.02.2015
(51) Int. Cl.: G01N 27/327, G01N 33/543, B01L 3/00, G01N 27/74

(54) **Method and device for detection and quantification of analytes**
Verfahren und Vorrichtung zum Nachweis und zur Quantifizierung von Analyten
Procédé et dispositif de détection et de quantification d'analytes

(43) Date of publication of application: 24.08.2016
(73) Proprietor: Fundacion Tekniker, 20600 Eibar (Guipuzkoa) (ES)
(72) Inventor: Merino, Santos, 20600 Eibar (Gipuzkoa) (ES); Eletxigerra, Unai, 20600 Eibar (Gipuzkoa) (ES); Juarros, Aritz, 20600 Eibar (Gipuzkoa) (ES); Martínez, Josu, 20600 Eibar (Gipuzkoa) (ES)
(74) Representative: Balder IP Law, S.L.

(56) References cited:
- WO-A1-2007/064635
- WO-A2-2008/098236
- US-A1- 2011 129 931
- ROSSIER J S ET AL: "GRAVI: Robotized Microfluidics for Fast and Automated Immunoassays in Low Volume", JOURNAL OF THE ASSOCIATION FOR LABORATORY AUTOMATION, ELSEVIER, vol. 13, no. 6, 1 December 2008 (2008-12-01), pages 322-329, XP025686863, ISSN: 1535-5535, DOI: 10.1016/J.JALA.2008.09.001 [retrieved on 2008-11-24]
- BERTI F ET AL: "Microfluidic-based electrochemical genosensor coupled to magnetic beads for hybridization detection", TALANTA, ELSEVIER, AMSTERDAM, NL, vol. 77, no. 3, 15 January 2009 (2009-01-15), pages 971-978, XP025760491, ISSN: 0039-9140, DOI: 10.1016/J.TALANTA.2008.07.064 [retrieved on 2008-08-14]
- JIN-WOO CHOI ET AL: "Development and Characterization of Microfluidic Devices and Systems for Magnetic Bead-Based Biochemical Detection", BIOMEDICAL MICRODEVICES, KLUWER, DORDRECHT, NL, vol. 3, no. 3, 1 September 2001 (2001-09-01), pages 191-200, XP002374416, ISSN: 1387-2176, DOI: 10.1023/A:1011490627871

## Description

### FIELD OF THE INVENTION

The present invention relates to a microfluidic device for rapid, ultrasensitive and in situ detection and quantification of different target analytes such as clinically important biomarkers in food or agricultural samples, human or animal fluids, or any environmental complex matrices. The invention also relates to a method of using said device.

### BACKGROUND OF THE INVENTION

There are several approaches devised to separate and detect a biomolecule of interest from complex samples in a microfluidic environment, such a human fluids.

Patent application US 2006/0257958 A1 discloses a microfluidic chip for the detection of target analytes in environmental, food, animal or clinical body fluid samples. Detection using the microfluidic chip is based on a sandwich immunoassay according to which target analytes present in a complex sample are allowed to interact with capture reagent-magnetic beads in a first position. The resulting complexes are moved to a second position, by means of an external magnetic field, and along the way, the analytes are thus purified and concentrated. At the second position the captured target analytes on the surface of the magnetic beads interact with secondary or "reporter" reagent, such as fluorescent dye-, fluorophore-, fluorescent protein-, quantum dot ("QD")-, nanoparticle-("NP"), colloidal gold-, or enzyme-conjugated antibodies, nucleic acid aptamers or fluorescence resonance energy transfer ("FRET") reagents such as FRET-aptamers or molecular beacons. In effect, a mobile or "rolling" sandwich assay is formed on the magnetic bead's surface. These reactants on the magnetic bead are further translated by the external magnetic field to a third position, which is a transparent miniature detection window for viewing of the resulting fluorescence or other visible reactions.

This microfluidic chip uses magnetic particles to trap the problem molecule and an external magnetic field to manipulate the sample along the chip allowing its manipulation, purification, and subsequent detection in different zones of the chip.

However, the sandwich immunoassay is done within the microfluidic chip increasing the need for more components and complicating its structure, like the microfluidic system which is complex. These aspects have a negative impact on the final cost of the microfluidic chip, the complexity of the method of using the chip and the cost of each measure. A further shortcoming of the assay can be seen in that the detection is based on optical measurements through a detection window, such as fluorescence, which needs a more complex and expensive detection hardware (light sources, filters, detection devices,...) rising the global cost of the system and impeding its miniaturization and consequently its use as point-of-care (POC) device.

ROSSIER J S ET AL: "GRAVI: Robotized Microfluidics for Fast and Automated Immunoassays in Low Volume", JOURNAL OF THE ASSOCIATION FOR LABORATORY AUTOMATION, ELSEVIER, vol. 13, no. 6, 1 December 2008 (2008-12-01), pages 322-329,ISSN: 1535-5535, DOI: 10.1016/J.JALA.2008.09.001 discloses a device for detecting and quantifying an analyte in a sample comprising a disposable cartridge with a measure chamber equipped with a working, reference and counter-electrode. A magnet is provided for attracting and retaining magnetic beads on the surface of the working electrode.

WO2007/064635 A1 discloses a microfluidic device in which a chamber and an associated magnet are used for purifying/washing magnetic beads prior to detection in a separate chamber. It further discloses that the washing chamber comprises a separate outlet.

WO2008/098236 A2 discloses the use of two electromagnets for manipulating magnetic beads. In view of the above there exists a need in the state of the art of providing an improved microfluidic device and method for the detection of a wide variety of analytes from complex fluid samples which overcomes at least part of these limitations.

### SUMMARY OF THE INVENTION

The present invention provides a device for detecting and quantifying an analyte in a complex sample which comprises: a disposable cartridge comprising: a microfluidic inlet for loading the sample to be tested, previously prepared, and therefore comprising the herein referred to as the reporter probe - analyte - capture probe - magnetic bead complexes, or for loading buffers, into the disposable cartridge; a purifying chamber for purifying the reporter probe - analyte - capture probe - magnetic bead complexes; a first microfluidic channel connecting the sample microfluidic inlet and the purifying chamber; a measure chamber comprising a chip comprising a working electrode, a reference electrode and a counter-electrode; a first waste microfluidic outlet for discharging residues derived from the purification of the reporter probe - analyte - capture probe - magnetic bead complexes carried out in the purifying chamber; a second waste microfluidic outlet for discharging waste products involved in the measuring process of the reporter probe - analyte - capture probe - magnetic bead complexes carried out in the measure chamber; a second microfluidic channel connecting purifying chamber and measure chamber; a first waste microfluidic channel connecting the second microfluidic channel to the first waste microfluidic outlet for discharging residues derived from the purification of the reporter probe - analyte - capture probe - magnetic bead complexes carried out in the purifying chamber; a second waste microfluidic channel connecting the measure chamber to the second waste fluid microfluidic outlet, for discharging residues involved in the measuring process of the reporter probe - analyte - capture probe - magnetic bead complexes carried out in the measure chamber; two external electromagnets situated one in front of the other, each on opposite sides of the purifying chamber which can be operated in a synchronized manner for retaining, or releasing or shaking the reporter probe - analyte - capture probe - magnetic bead complexes in the purifying chamber; a permanent magnet located under the working electrode of the measurement chamber, to attract and retain the reporter probe - analyte - capture probe - magnetic bead complexes on the surface of the working electrode of the chip; a potentiostat for carrying out the electrochemical detection and quantification of the analyte by electrochemical means; and an external microfluidic system.

The microfluidic device of the invention is a versatile tool that allows performing detection and quantification of a vast variety of analytes in complex samples such as environmental, food, animal or body fluid samples. The device of the invention has simplified microfluidics and structure with the subsequent fabrication cost reduction and assay performance enhancement. The reporter probe - analyte - capture probe - magnetic bead complexes preparation is advantageously carried out outside the disposable cartridge which allows a simpler cartridge design, with fewer chambers, fewer channels (and thus lower fabrication cost than other known cartridges of the art) and a reduced microfluidic actuation system (pump and valve system). Thus, just one prepared sample, containing the reporter probe - analyte - capture probe - magnetic bead complex, is introduced in the cartridge in the first place, and all the operations required are performed to the reporter probe - analyte - capture probe - magnetic bead complex contained therein, resulting in a sequential process, giving little room for errors and repetitiveness issues.

In a particular embodiment, the device of the invention further comprises a mechanical system to pick up the disposable cartridge, and place it in its proper position in the device, and make the microfluidic and electric connections.

In another particular embodiment the device of the invention further comprises a control system for the automatization of all components of the device.

In another particular embodiment the device of the invention further comprises a display.

In another particular embodiment the device of the invention further comprises an outer casing.

The external microfluidic system comprises in a particular embodiment one or more microfluidic channels, one or more microfluidic connections, one or more pumps and one or more valves for manipulating the sample, buffers and residues.

The present invention also provides a method of using the device of the present invention for the detection and quantification of an analyte in a sample comprising the steps of: (a) loading the sample to be analyzed, magnetic beads coated with a capture probe and a reporter probe in a container to form a suspension comprising reporter probe - analyte - capture probe - magnetic bead complexes; (b) introducing the prepared sample (a resulting suspension) through the sample microfluidic inlet in the purifying chamber of the disposable cartridge; (c) purifying the reporter probe - analyte - capture probe - magnetic bead complexes in the purifying chamber by operating in a synchronized manner the two external electromagnets for retaining, releasing and shaking the reporter probe - analyte - capture probe - magnetic bead complexes present in the sample in the purifying chamber, (d) forwarding the purified reporter probe - analyte - capture probe - magnetic bead complexes resulting from previous step (c) through the second microfluidic channel to the measure chamber, (e) operating the permanent magnet to retain the reporter probe - analyte - capture probe - magnetic bead complexes on the working electrode surface of the chip, (f) detecting and quantifying the analyte (e.g. reporter probe - analyte - capture probe - magnetic bead complexes) by electrochemical means.

The method of the invention allows the detection and quantification of diverse analytes which can be present in complex samples with high efficacy and sensitivity. The method can be advantageously carried out in less steps than other known methods in the art, since the magnetic beads with the capture probes, the reporter probes and the samples eventually containing the analytes are put together in contact so that the reporter probe - analyte - capture probe - magnetic bead complexes are built in only one step. In addition detection and quantification is based on electrochemical means, a very accurate and sensitive detection technique, very cost effective and highly miniaturizable and portable, all of them essential characteristics in a POC device.

According to a particular embodiment the purification of the reporter probe - analyte - capture probe - magnetic bead complexes in the purifying chamber comprises the steps of: (i) operating the two external electromagnets for retaining the reporter probe - analyte - capture probe - magnetic bead complexes on at least part of the inner surface of the purifying chamber, (ii) letting the rest of the sample present in the purifying chamber flow off, via the microfluidic channel and the first waste microfluidic outlet, (iii) loading buffer solution through the inlet into the purifying chamber, (iv) switching off the two external electromagnets for releasing the reporter probe - analyte - capture probe - magnetic bead complexes and re-suspending them in the buffer, (v) operating the two external electromagnets for shaking the reporter probe - analyte - capture probe - magnetic bead complexes in the purifying chamber, and (vi) optionally repeating steps (i) to (v) once or more times.

In a particular embodiment the step of detecting and quantificating the analyte by electrochemical means is carried out by voltammetry, amperometry or impedance measurement techniques.

More particularly step (f) of the method of using the device of the present invention comprises: connecting the chip to the potentiostat by electrical contacts; filling the measure chamber with buffer containing an electron transfer mediator substance; monitoring the initial electrochemical signal, launching the electrochemical detection procedure using the potentiostat; adding the substrate to trigger a redox reaction; recording electrochemical signal shift produced by a redox reaction; translating resulting electrochemical signal shift in a numeric value on the display.

### BRIEF DESCRIPTION OF THE FIGURES

Fig. 1: shows a schematic representation of a device of the present invention with a perspective view of the disposable cartridge and some of its components.
Fig. 2: shows a perspective view of the separated two halves of the disposable cartridge of the present invention before they build together the cartridge.

### DETAILED DESCRIPTION OF THE INVENTION

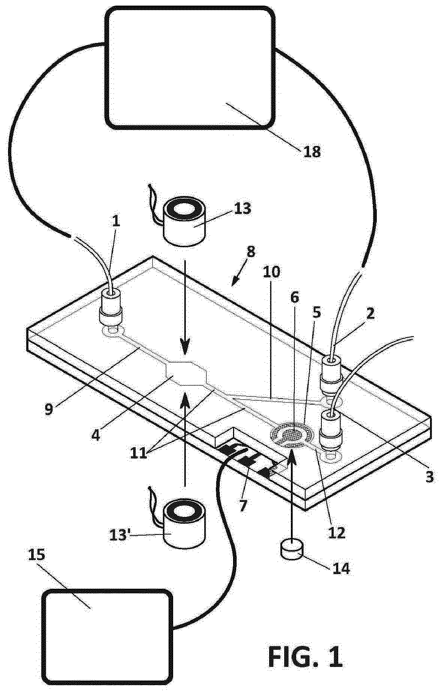

Figure 1 shows a schematic representation of a device for detecting and quantifying an analyte in a sample according to a particular embodiment of the present invention. The device comprises a disposable cartridge 8, two external (and opposed) electromagnets 13 and 13', a permanent magnet 14, a potentiostat 15, and an external microfluidic system 18.
The external microfluidic system 18 comprises one or more microfluidic channels, one or more microfluidic connections, one or more pumps and one or more valves for manipulating the sample, buffers, and fluid residues. In a particular embodiment the external microfluidic system 18 comprises two pumps. In another particular embodiment the external microfluidic system 18 comprises a selection valve and an injection valve.

The disposable cartridge 8 comprises a microfluidic inlet 1, a first microfluidic channel 9, a purifying chamber 4, a measure chamber 5 comprising a chip 6, a second microfluidic channel 11, a first waste microfluidic channel 10, a first waste microfluidic outlet 2, a second waste microfluidic channel 12, and a second waste microfluidic outlet 3.
Both waste microfluidic outlets 2 and 3 are connected to a waste container not shown in Fig. 1.
In the aforementioned cartridge 8 the microfluidic inlet is for loading the sample to be analyzed. The sample is prepared outside the device in a suitable container, such as a vial, and comprises after its preparation, provided that the analyte to be detected is present, the herein referred to as reporter probe - analyte - capture probe - magnetic bead complexes. The microfluidic inlet 1 is also for loading buffer into the cartridge. The sample comprising the reporter probe - analyte - capture probe - magnetic bead complexes is prepared by putting into contact a sample containing an analyte to be detected and quantified, magnetic beads coated with a capture probe which binds the analyte of interest through affinity mechanisms and a reporter probe which also binds said analyte of interest through affinity mechanisms. The sample comprising the reporter probe - analyte - capture probe - magnetic bead complexes flows from the inlet 1 through the first microfluidic channel 9 until the purifying chamber 4.
In the aforementioned cartridge 8 the purifying chamber 4 is for purifying the reporter probe - analyte - capture probe - magnetic bead complexes from the rest of the components of the sample. This is achieved with the help of the two external electromagnets 13 and 13' which can be operated in a synchronized manner for retaining, or releasing or shaking the reporter probe - analyte - capture probe - magnetic bead complexes present in the purifying chamber 4. First the magnets are operated to retain the complexes at least on part of the inner surface of the purifying chamber 4. Subsequently, the rest of the sample which is not retained flows through the microfluidic channels 11, 10, and finally outside the cartridge 8 through the first waste microfluidic outlet 2. This first waste microfluidic outlet 2 is for discharging residues derived from the purification of the reporter probe - analyte - capture probe - magnetic bead complexes carried out in the purifying chamber 4. Fresh buffer solution may be again loaded through inlet 1 into the purifying chamber 4. The two external electromagnets 13 and 13' are then switched off for releasing the reporter probe - analyte - capture probe - magnetic bead complexes in the purifying chamber 4 rendering a suspension of the complexes in the fresh buffer. The two external electromagnets 13 and 13' are then operated in a synchronized manner for shaking the suspension. Again the two external electromagnets 13 and 13' can be operated to retain the complexes at least on part of the inner surface of the purifying chamber 4. Subsequently, the rest of the buffer which is not retained flows through the microfluidic channels 11 and 10 and finally outside the cartridge 8 through the first waste microfluidic outlet 2. Fresh buffer solution is loaded then through inlet 1 into the purifying chamber 4. The two external electromagnets 13 and 13' are then switched off for releasing the reporter probe - analyte - capture probe - magnetic bead complexes in the purifying chamber 4 rendering a suspension of the complexes in the fresh buffer. The two external electromagnets 13 and 13' are then operated in a synchronized manner for shaking the suspension.

This combination of steps for the purification of the complexes can be repeated once or more times until the desired degree of purity is achieved.

In the aforementioned cartridge 8 the microfluidic channel 11 is for conducting the purified reporter probe - analyte - capture probe - magnetic bead complexes to the a measure chamber 5. The measure chamber 5 is for measuring the analyte and comprises a chip 6 comprising a working electrode, a reference electrode and a counter-electrode. The electrodes may be made according to a particular embodiment of gold.

A second waste microfluidic channel 12 is for connecting the measure chamber 5 to a second waste microfluidic outlet 3, for discharging residues involved in the measuring and sensing process of the reporter probe - analyte - capture probe - magnetic bead complexes carried out in the measure chamber 5.

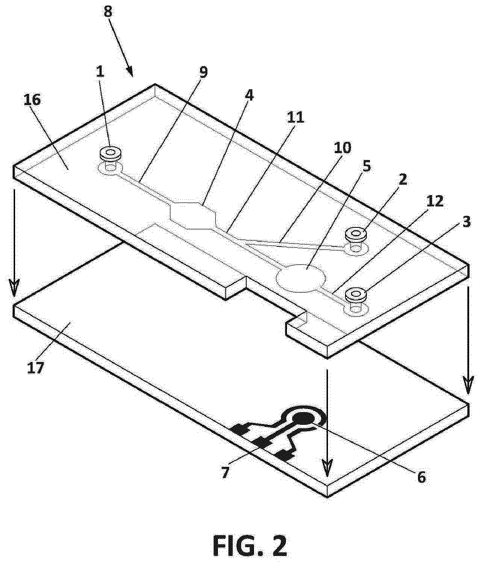

As schematically shown in Fig. 2, the disposable cartridge 8 is fabricated from two halves 16 and 17 typically composed of disposable plastic. The two halves 16 and 17 can be joined by way of heat (such as fusion, laser, welding, ultrasound, microwaves, solvents or other means of melting the halves together), or adhesives (such as pressure sensitive adhesives). In Fig. 2, the chip 6 comprising a working electrode, a reference electrode and a counter-electrode and the electrical contacts 7 can be seen. The chip is imprinted on the lower half of the cartridge by means of screen printing of thin film deposition or any other metal imprinting technique. The microfluidic channels are carved in the top half of the cartridge by any polymer fabrication techniques (mechanization, injection, hot embossing, laser, etc.).
The electrical contacts 7 are for connecting the chip 6 and the potentiostat 15 (not shown in Fig. 2), which is the equipment for carrying out the electrochemical detection and quantification of the analyte by electrochemical means.

The permanent magnet 14 is for retaining the reporter probe - analyte - capture probe - magnetic bead complexes to allow its detection. As shown in Fig. 1 the permanent magnet 14 is situated under the working electrode and thus when the reporter probe - analyte - capture probe - magnetic bead complexes reach the measure chamber 5, they are retained by the influence of permanent magnet 14 covering thus only the working electrode.

The potentiostat 15, also shown in Fig.1, is for carrying out the electrochemical detection and quantification of the analyte by electrochemical means.

The device of the invention comprises further components which are not shown in Fig. 1: like a mechanical system to pick up the disposable cartridge 8 and place it in its proper position in the device, and make the microfluidic and electric connections; a control system for the automatization of all components of the device; a display for showing the instructions of use to the operator, the assay steps or the results of the analysis; or an outer casing for integrating all components.

A method of using the device of the invention allows for the detection and quantification of an analyte in a sample. The method only requires a short assay time, very small target substance amounts, even trace quantities, and exhibits high sensitivity. This method, hereinafter also referred to as the method of the invention comprises the following steps:
(a) loading the sample to be analyzed, magnetic beads coated with a capture probe and a reporter probe in a container to form a suspension comprising the reporter probe - analyte - capture probe - magnetic bead complexes,
(b) introducing the resulting suspension through a sample microfluidic inlet 1 in the purifying chamber 4 of a disposable cartridge 8;
(c) purifying the reporter probe - analyte - capture probe - magnetic bead complexes in the purifying chamber 4 by operating in a synchronized manner the two external electromagnets 13 and 13' for retaining, releasing and shaking the reporter probe - analyte - capture probe - magnetic bead complexes present in the sample in the purifying chamber 4,
(d) forwarding the purified reporter probe - analyte - capture probe - magnetic bead complexes resulting from previous step (c) through the second microfluidic channel 11 to the measure chamber 5,
(e) operating the permanent magnet 14 to retain the reporter probe - analyte - capture probe - magnetic bead complexes on the working electrode surface of the chip 6,
(f) detection and quantification of the analyte (e.g. reporter probe - analyte - capture probe - magnetic bead complexes) by electrochemical means.

Further advantages of the method of the invention are its high accuracy and reliability.

The samples that can be analyzed in accordance with embodiments of the invention include, without limitation, plasma, serum, urine, saliva, tear, whole blood, blood analogs, and liquid solution from dilution of solid biological matter or other biological fluids, such as milk, meat, fruit, vegetable, or any environmental sample potentially containing the analyte to be tested such as soil or water.

Analytes, without limitation, that can be detected and quantified in accordance with embodiments of the invention include, without limitation, proteins, protein fragments, antigens, antibodies, antibody fragments, peptides, DNA, RNA, DNA fragments, RNA fragments, or any other molecular target of interest from complex samples.

Any magnetic beads that respond to a magnetic field may be employed in the devices and methods of the invention. Desirable beads are those that have surface chemistry that can be chemically or physically modified, e.g., by chemical reaction, physical adsorption, entanglement, or electrostatic interaction.

Capture probes can be bound to magnetic beads coating them by any means known in the art. Examples include chemical reaction, physical adsorption, entanglement, or electrostatic interaction. How the capture probe binds to the magnetic bead will depend on the nature of the analyte targeted. Examples of capture probes in accordance with embodiments of the invention include, without limitation, proteins (such as antibodies), DNA, RNA, PNA or similar nucleic acid based probes for DNA or RNA, aptamers, molecularly imprinted polymers or any other molecule capable of binding efficiently and specifically to the analyte of interest.

Reporter probes are in principle any chemical species capable of binding efficiently and specifically to the analyte of interest and are labeled with a molecule capable of producing a redox reaction, usually a redox enzyme such as horseradish peroxidase or alkaline phosphatase. Examples in accordance with embodiments of the invention include, without limitation, proteins (such as antibodies), DNA, RNA, PNA or similar nucleic acid based probes for DNA or RNA, aptamers, molecularly imprinted polymers or any other molecule capable of binding efficiently and specifically to the analyte of interest.

By "specifically binding" to the analyte is meant binding analytes by a specified mechanism, e. g., antibody-antigen interaction, ligand-receptor interaction, nucleic acid complementarity, protein-protein interaction, charge- charge interaction, and hydrophobic-hydrophobic or hydrophilic-hydrophilic interactions. "Efficiently" means that the strength of the bond is enough to prevent detachment by the flow of fluid present when analytes are bound, although individual analytes may occasionally detach under normal operating conditions.

One remarkable advantage of the method of the invention can be seen in that the samples to be tested containing the analyte are prepared in one single, simple step, outside the disposable cartridge of the device used for carrying out the method of the present invention. Accordingly, the reporter probe and magnetic beads coated with capture probes are provided together in an adequate container such as a vial. As long as no target molecule is present, these two components remain separated. At the beginning of the assay, the sample is added to this vial, and if the analyte is present in the sample, the reporter probe - analyte - capture probe - magnetic bead complex is thus obtained. The reporter probe and the capture probe can be readily selected by the skilled person in the art depending on the analyte to be detected in each case. The molecule capable of producing a redox reaction for labeling the reporter probe and the magnetic bead can also be selected by the skilled person in each case without the need of any inventive step.

According to a particular embodiment the analyte to be detected is an antigen. Magnetic beads are coated with a capture probe which is a first antigen specific monoclonal antibody (Ab1) binding to a first region (first epitope) of the antigen; the reporter probe is a second antigen specific polyclonal antibody (Ab2) binding to a second region of the antigen and labeled with a redox enzyme.

The suspension of complexes is introduced through the sample microfluidic inlet 1 in the purifying chamber 4 of the disposable cartridge 8. In the purifying chamber 4 the reporter probe - analyte - capture probe - magnetic bead complexes are purified, thus separated from the rest of the sample. In accordance with embodiments of the invention the purification is done as follows with the help of the two external electromagnets 13 and 13'. The two external electromagnets 13 and 13' are operated for retaining the reporter probe - analyte - capture probe - magnetic bead complexes on at least part of the inner surface of the purifying chamber 4. While the complexes are retained, the rest of the sample of no interest flows off via the microfluidic channel 10 and through the first waste microfluidic outlet 2 outside the cartridge for example into a waste container. A buffer solution is loaded through the inlet 1 into the purifying chamber 4, and then the two external electromagnets 13 and 13' are switched off for releasing the reporter probe - analyte - capture probe - magnetic bead complexes from the inner wall, resulting in a suspension of the complexes in the fresh buffer. The two external electromagnets are operated for shaking the reporter probe - analyte - capture probe - magnetic bead complexes in suspension present in the purifying chamber 4, which helps dispersing the complexes in the buffer and favors its cleaning process. The previous steps may then be repeated to further increase the purification degree of the complexes.
Thus, the two external electromagnets 13 and 13' may again be operated for retaining the reporter probe - analyte - capture probe - magnetic bead complexes on at least part of the inner surface of the purifying chamber 4. While the complexes are retained, the rest of the buffer flows off via the microfluidic channel 10 and through the first waste microfluidic outlet 2 outside the cartridge for example into a waste container. Fresh buffer solution is loaded through the inlet 1 into the purifying chamber 4, and then the two external electromagnets 13 and 13' are switched off for releasing the reporter probe - analyte - capture probe - magnetic bead complexes from the inner wall, resulting in a suspension of the complexes in the fresh buffer. The two external electromagnets are operated for shaking the reporter probe - analyte - capture probe - magnetic bead complexes in suspension present in the purifying chamber 4.
When the complexes are considered to be pure enough a suspension comprising them is forwarded to measure chamber 5 through microfluidic channel 11.

Detection and quantification of the analyte (e.g. reporter probe - analyte - capture probe - magnetic bead complexes) is carried out by electrochemical means with a potentiostat 15. Voltammetry, amperometry or impedance measurement techiques can be used. These techniques are based on the application of an electrical potential and the measurement of the produced electrical current, such as voltammetries (cyclic voltammetry, linear sweep voltammetry, differential pulse voltammetry, or square wave voltammetry), amperometries (chrono amperometry) or impedance measurements.
Despite the fact that a great variety of different electrochemical measurement techniques fit the needs of the detection procedure, according to a particular embodiment amperometric measurement is used.

In accordance with embodiments method of the invention comprises the steps of:
- Connecting the chip 6 to the potentiostat 15 by electrical contacts 7;
- Filling the measure chamber 5 with buffer containing an electron transfer mediator substance,
- Monitoring the initial electrochemical signal, launching the electrochemical detection procedure using the potentiostat,
- Adding the substrate to trigger a redox reaction,
- Recording electrochemical signal shift produced by a redox reaction,
- Translating resulting electrochemical signal shift in a numeric value on the display.
When complexes reach the measure chamber 5 the permanent magnet 14 retains the reporter probe - analyte - capture probe - magnetic bead complexes to allow its detection. The permanent magnet 14 is situated under the working electrode and thus when the reporter probe - analyte - capture probe - magnetic bead complexes reach the measure chamber they are retained by magnet 14 on the working electrode. The buffer is consequently eliminated via microfluidic channel 12 and through waste microfluidic outlet 3, for example, to a waste container. Then, the measure chamber 5 is filled with buffer comprising an electron transfer mediator substance, which is introduced through the microfluidic inlet 1.
The electron transfer mediator substances that can be used are active charge-carrier substances that act as intermediates, enabling electrical contacting between the electrode surface and the active center of redox enzymes, such as ferrocene or hydroquinone.
The substrate that can be used to trigger the redox reaction is any chemical species upon which the enzyme acts, catalyzing chemical reactions involving the substrate. The substrate is specific to the enzyme used to label the reporter probe, for example hydrogen peroxide for the horseradish peroxidase.

To perform an amperometric measurement according to a particular embodiment of the invention, the reporter probe - analyte - capture probe - magnetic bead complex is captured onto the measurement chip working electrode by means of the permanent magnet situated exactly under. The measurement chamber is then filled with a buffer solution (like phosphate solution), containing the electrochemical mediator substance (hydroquinone, for example), but not the enzymatic substrate. The potentiostat, connected to the chip, is switched on and the amperometric signal (electrical current) is monitored while applying an electrical potential (vs. the reference electrode). After obtaining a stable signal (baseline), a buffer change is performed. This new buffer is the same used in the recording of a baseline, but with the addition of a given concentration the enzymatic substrate. When this new buffer containing the enzymatic substrate fills the measurement chamber, the enzymatic redox reaction is triggered and a current change induced. This current change is proportional to the amount of enzyme captured on the magnetic beads which at the same time is proportional to the analyte concentration present in the sample. Thus, the monitored current change is used to calculate the analyte concentration present in the sample. This electrical signal is translated to a numerical value and presented to the user via the display situated in the outer casing of the device.

## Claims

1. A device for detecting and quantifying an analyte in a sample which comprises:
- a disposable cartridge (8) comprising:
a microfluidic inlet (1) for loading the sample comprising reporter probe, analyte, capture probe and magnetic bead complexes, or for loading buffers, into the cartridge
a purifying chamber (4) for purifying the reporter probe, analyte, capture probe and magnetic bead complexes
a first microfluidic channel (9) connecting the sample microfluidic inlet (1) and the purifying chamber (4),
a measure chamber (5) comprising a chip (6) comprising a working electrode, a reference electrode and a counter-electrode,
a first waste microfluidic outlet (2) for discharging residues derived from the purification of the reporter probe, analyte, capture probe and magnetic bead complexes carried out in the purifying chamber (4),
a second waste microfluidic outlet (3) for discharging residues involved in the measuring and sensing process of the reporter probe, analyte, capture probe and magnetic bead complexes carried out in the measure chamber (5),
a second microfluidic channel (11) connecting purifying chamber (4) and measure chamber (5),
a first waste microfluidic channel (10) connecting the second microfluidic channel (11) to the first waste microfluidic outlet (2) for discharging residues derived from the purification of the reporter probe, analyte, capture probe and magnetic bead complexes carried out in the purifying chamber (4),
a second waste microfluidic channel (12) connecting the measure chamber (5) to the second waste fluid microfluidic outlet (3), for discharging residues involved in the measuring and sensing process of the reporter probe, analyte, capture probe and magnetic bead complexes carried out in the measure chamber (5).
- two external electromagnets (13, 13') situated one in front of the other, each on opposite sides of the purifying chamber (4) which can be operated in a synchronized manner for retaining, or releasing or shaking the reporter probe, analyte, capture probe and magnetic bead complexes present in the purifying chamber (4),
- a permanent magnet (14) located under the working electrode of the measurement chamber (5), to attract and retain the reporter probe, analyte, capture probe and magnetic bead complexes on the surface of the working electrode of the chip (6),
- a potentiostat (15) for carrying out the electrochemical detection and quantification of the analyte by electrochemical means, and
- an external microfluidic system (18).

2. The device of claim 1, further comprising a mechanical system to pick up the disposable cartridge (8), place it in its proper position in the device, and make the microfluidic and electric connections.

3. The device of claim 1 or 2, further comprising a control system for the automatization of all components of the device.

4. The device according to any one of claims 1 to 3, further comprising a display.

5. The device according to any one of claims 1 to 4, further comprising an outer casing.

6. The device according to any one of claims 1 to 5, wherein the external microfluidic system (18) comprises one or more microfluidic channels, one or more microfluidic connections, one or more pumps and one or more valves for manipulating the sample, buffers, and fluid wastes.

7. A method of using the device according to any one of the preceding claims, for the detection and quantification of an analyte in a sample comprising the following steps:
a) loading the sample to be analyzed, magnetic beads coated with a capture probe and a reporter probe in a container to form a suspension comprising reporter probe, analyte, capture probe and magnetic bead complexes,
b) introducing the resulting suspension through a microfluidic inlet (1) in the purifying chamber (4) of a disposable cartridge (8);
c) purifying the reporter probe, analyte, capture probe and magnetic bead complexes in the purifying chamber (4) by operating in a synchronized manner the two external electromagnets (13, 13') for retaining, releasing and shaking the reporter probe, analyte, capture probe and magnetic bead complexes present in the sample in the purifying chamber (4),
d) forwarding the purified reporter probe, analyte, capture probe and magnetic bead complexes resulting from previous step c) through the second microfluidic channel (11) to the measure chamber (5),
e) operating the permanent magnet (14) to retain the reporter probe, analyte, capture probe and magnetic bead complexes on the working electrode surface of the chip (6),
f) detection and quantification of the analyte by electrochemical means.

8. A method according to claim 7, wherein the purifying of the reporter probe, analyte, capture probe and magnetic bead complexes in the purifying chamber (4) comprises the steps of
(i) operating the two external electromagnets (13, 13') for retaining the reporter probe, analyte, capture probe and magnetic bead complexes on at least part of the inner surface of the purifying chamber (4),
(ii) letting the rest of the sample present in the purifying chamber (4) flow off, via microfluidic channel (10) and first waste microfluidic outlet (2),
(iii) loading a buffer solution through the microfluidic inlet (1) into the purifying chamber (4),
(iv) switching off the two external eletromagnets (13, 13') for releasing the reporter probe, analyte, capture probe and magnetic bead complexes and resuspending them in buffer,
(v) operating the two external electromagnets (13, 13') for shaking the reporter probe, analyte, capture probe and magnetic bead complexes in the buffer in the purifying chamber (4),
(vi) optionally repeating steps (i) to (v).

9. A method according to claim 7 or 8, wherein the detection and quantification of the analyte by electrochemical means is carried out by voltammetry, amperometry or impedance measurement.

10. A method according to claim 9, which comprises:
- connecting the chip (6) to the potentiostat (15) by electrical contacts (7)
- filling the measure chamber (5) with buffer containing an electron transfer mediator substance,
- monitoring the initial electrochemical signal, launching the electrochemical detection procedure using the potentiostat,
- adding the substrate to trigger a redox reaction,
- recording electrochemical signal shift produced by a redox reaction,
- translating resulting electrochemical signal shift in a numeric value on the display.

## Patentansprüche

1. Eine Vorrichtung zum Nachweisen und Quantifizieren eines Analyten in einer Probe, umfassend:
- eine Einwegkartusche (8), umfassend:
einen Mikrofluidikeinlass (1) zum Laden der Probe, umfassend eine Reportersonde, einen Analyten, eine Einfangsonde und Magnetkügelchenkomplexe, oder zum Laden von Puffern in die Kartusche,
eine Aufreinigungskammer (4) zum Aufreinigen der Reportersonde, des Analyten, der Einfangsonde und der Magnetkügelchenkomplexe,
einen ersten Mikrofluidkanal (9), der den Mikrofluidikprobeneinlass (1) und die Aufreinigungskammer (4) verbindet,
eine Messkammer (5), umfassend einen eine Arbeitselektrode, eine Referenzelektrode und eine Gegenelektrode umfassenden Chip (6),
einen ersten Mikrofluidikabfallauslass (2) zum Abführen von Rückständen aus der in der Aufreinigungskammer (4) durchgeführten Aufreinigung der Reportersonde, des Analyten, der Einfangsonde und der Magnetkügelchenkomplexe,
einen zweiten Mikrofluidikabfallauslass (3) zum Abführen von Rückständen, die an dem in der Messkammer (5) durchgeführten Mess- und Nachweisprozess der Reportersonde, des Analyten, der Einfangsonde und der Magnetkügelchenkomplexe beteiligt sind,
einen zweiten Mikrofluidikkanal (11), der die Aufreinigungskammer (4) und die Messkammer (5) verbindet,
einen ersten Mikrofluidikabfallkanal (10), der den zweiten Mikrofluidikkanal (11) und den ersten Mikrofluidikabfallauslass (2) zum Abführen von Rückständen aus der in der Aufreinigungskammer (4) durchgeführten Aufreinigung der Reportersonde, des Analyten, der Einfangsonde und der Magnetkügelchenkomplexe verbindet,
einen zweiten Mikrofluidikabfallkanal (12), der die Messkammer (5) und den zweiten Mikrofluidikabfallauslass (3) zum Abführen von Rückständen, die an dem in der Messkammer (5) durchgeführten Mess- und Nachweisprozess der Reportersonde, des Analyten, der Einfangsonde und der Magnetkügelchenkomplexe beteiligt sind, verbindet,
- zwei einander gegenüberliegende externe Elektromagneten (13, 13') jeweils auf gegenüberliegenden Seiten der Reinigungskammer (4) angeordnet, die in synchroner Weise zum Zurückhalten, zum Freisetzen oder Schütteln der in der Aufreinigungskammer (4) vorhandenen Reportersonde, des Analyten, der Einfangsonde und der Magnetkügelchenkomplexe betrieben werden können,
- einen Permanentmagnet (14), der unter der Arbeitselektrode der Messkammer (5) angeordnet ist, um die Reportersonde, den Analyten, die Einfangsonde und die Magnetkügelchenkomplexe auf der Oberfläche der Arbeitselektrode des Chips (6) anzuziehen und zurückzuhalten,
- ein Potentiostat (15) zur Durchführung des elektrochemischen Nachweises und Quantifizierung des Analyten durch elektrochemische Mittel, und
- ein externes Mikrofluidiksystem (18).

2. Die Vorrichtung nach Anspruch 1, ferner umfassend ein mechanisches System zum Aufnehmen der Einwegkartusche (8), zum Platzieren ihrer richtigen Position in der Vorrichtung und zum Herstellen der mikrofluidischen und elektrischen Verbindungen.

3. Die Vorrichtung nach Anspruch 1 oder 2, ferner umfassend ein Steuersystem zur Automatisierung aller Komponenten der Vorrichtung.

4. Die Vorrichtung entsprechend einem der Ansprüche 1 bis 3, ferner umfassend eine Anzeige.

5. Die Vorrichtung entsprechend einem der Ansprüche 1 bis 4, ferner umfassend ein äußeres Gehäuse.

6. Die Vorrichtung nach einem der Ansprüche 1 bis 5, wobei das externe Mikrofluidiksystem (18) einen oder mehrere Mikrofluidikkanäle, eine oder mehrere Mikrofluidikverbindungen, eine oder mehrere Pumpen und ein oder mehrere Ventile zum Manipulieren der Probe, des Puffers und der Flüssigkeitsabfälle umfasst.

7. Ein Verfahren zur Verwendung der Vorrichtung entsprechend einem der vorhergehenden Ansprüche zum Nachweis und zur Quantifizierung eines Analyten in einer Probe, umfassend die folgenden Schritte:
a) Laden der zu analysierenden Probe mit einer Einfangsonde und einer Reportersonde beschichtete magnetische Kügelchen in einen Behälter, um eine Suspension zu bilden, die eine Reportersonde, einen Analyten, eine Einfangsonde und Magnetkügelchenkomplexe umfasst,
b) Einführen der resultierenden Suspension in die Reinigungskammer (4) einer Einwegkartusche (8) durch einen Mikrofluidikeinlass (1);
c) Aufreinigen der Reportersonde, des Analyten, der Einfangsonde und der Magnetkügelchenkomplexe in der Aufreinigungskammer (4) durch Betreiben der beiden äußeren Elektromagnete (13, 13') in synchroner Weise zum Zurückhalten, Freisetzen und Schütteln der in der Probe vorhandenen Reportersonde, des Analyten, der Einfangsonde und der Magnetkügelchenkomplexe in der Aufreinigungskammer (4),
d) Weiterleiten der aus dem vorherigen Schritt c) resultierenden, aufgereinigten Reportersonde, Analyten, Einfangsonde und Magnetkügelchenkomplexe durch den zweiten Mikrofluidikkanal (11) in die Messkammer (5),
e) Betreiben des Permanentmagneten (14) zum Zurückhalten der Reportersonde, Analyten, Einfangsonde und Magnetkügelchenkomplexe auf der Arbeitselektrodenoberfläche des Chips (6),
f) Nachweisen und Quantifizieren des Analyten durch elektrochemische Mittel.

8. Ein Verfahren entsprechend Anspruch 7, wobei das Aufreinigen der Reportersonde, des Analyten, der Einfangsonde und der Magnetkügelchenkomplexe in der Aufreinigungskammer (4) die folgenden Schritte umfasst von
(i) Betreiben der beiden externen Elektromagneten (13, 13') zum Zurückhalten der Reportersonde, des Analyten, der Einfangsonde und der Magnetkügelchenkomplexe auf mindestens einem Bereich der inneren Oberfläche der Aufreinigungskammer (4),
(ii) Abfließen lassen des Rests der in der Reinigungskammer (4) vorhandenen Probe durch Mikrofluidikkanal (10) und den ersten Mikrofluidikabfallauslass (2),
(iii) Laden einer Pufferlösung in die Aufreinigungskammer (4) durch den Mikrofluidikeinlass (1),
(iv) Abschalten der beiden externen Elektromagneten (13, 13') zum Freisetzen der Reportersonde, des Analyten, der Einfangsonde und der Magnetkügelchenkomplexe und zum Resuspendieren derer in Puffer,
(v) Betreiben der beiden externen Elektromagnete (13, 13') zum Schütteln der Reportersonde, des Analyten, der Einfangsonde und der Magnetkügelchenkomplexe im Puffer in der Aufreinigungskammer (4),
(vi) wahlweise Wiederholen der Schritte (i) bis (v).

9. Ein Verfahren entsprechend Anspruch 7 oder 8, wobei der Nachweis und die Quantifizierung des Analyten durch elektrochemische Mittel durch Voltammetrie, Amperometrie oder Impedanzmessung erfolgen.

10. Ein Verfahren entsprechend Anspruch 9, das umfasst:
- Verbinden des Chips (6) mit dem Potentiostaten (15) durch elektrische Kontakte (7),
- Füllen der Messkammer (5) mit einem eine Elektronentransfermediatorsubstanz enthaltenden Puffer,
- Überwachen des initialen elektrochemischen Signals, Starten des elektrochemischen Nachweisverfahrens unter Verwendung des Potentiostaten,
- Hinzufügen des Substrats, um eine Redoxreaktion auszulösen,
- Aufzeichnung einer durch eine Redoxreaktion erzeugten elektrochemischen Signalverschiebung,
- Übersetzen der resultierenden elektrochemischen Signalverschiebung in einen numerischen Wert auf dem Display.

## Revendications

1. Dispositif pour détecter et de quantifier un analyte dans un échantillon, lequel dispositif comprend :
- une cartouche jetable (8) comprenant :
une entrée microfluidique (1) pour introduire l'échantillon comprenant des complexes sonde rapporteuse, analyte, sonde de capture et billes magnétiques, ou pour introduire des tampons, dans la cartouche,
une chambre de purification (4) pour purifier les complexes sonde rapporteuse, analyte, sonde de capture et billes magnétiques
un premier canal microfluidique (9) reliant l'entrée microfluidique d'échantillon (1) et la chambre de purification (4),
une chambre de mesure (5) comprenant une puce (6) comprenant une électrode de travail, une électrode de référence et une électrode auxiliaire,
une première sortie microfluidique de déchets (2) pour évacuer les résidus dérivés de la purification des complexes sonde rapporteuse, analyte, sonde de captures et billes magnétiques réalisée dans la chambre de purification (4),
une deuxième sorite microfluidique de déchets (3) pour évacuer les résidus impliqués dans le processus de mesure et de détection des complexes sonde rapporteuse, analyte, sonde de capture et billes magnétiques réalisé dans la chambre de mesure (5),
un deuxième canal microfluidique (11) reliant la chambre de purification (4) et la chambre de mesure (5),
un premier canal microfluidique de déchets (11) reliant le second canal microfluidique (11) et la première sortie microfluidique de déchets (2) pour évacuer des résidus dérivés de la purification des complexes sonde rapporteuse, analyte, sonde de capture et billes magnétiques réalisée dans la chambre de purification (4), un deuxième canal microfluidique de déchets (12) reliant la chambre de mesure (5) et la deuxième sortie microfuidique de déchets (3) pour évacuer des résidus impliqués dans le processus de mesure et de détection des complexes sonde rapporteuse, analyte, sonde de capture et billes magnétiques réalisé dans la chambre de mesure (5),
- deux électro-aimants externes (13, 13') disposés l'un en face de l'autre, chacun sur des côtés opposés de la chambre de purification (4), et qui peuvent être actionnés de façon synchronisée pour retenir, ou relâcher ou agiter les complexes sonde rapporteuse, analyte, sonde de capture et billes magnétiques présents dans la chambre de purification (4),
- un aimant permanent (14) situé sous l'électrode de travail de la chambre de mesure (5), pour attirer et retenir les complexes sonde rapporteuse, analyte, sonde de capture et billes magnétiques sur la surface de l'électrode de travail de la puce (6),
- et un potentiostat (15) pour réaliser la détection et la quantification électrochimiques de l'analyte par des moyens électrochimiques, et
- un system microfluidique externe (18).

2. Dispositif selon la revendication 1, comprenant en outre un système mécanique pour saisir la cartouche jetable (8), la placer dans sa position appropriée dans le dispositif, et réaliser les connexions microfluidiques et électriques.

3. Dispositif selon la revendication 1 ou 2, comprenant en outre un système de commande pour l'automatisation de tous les composants du dispositif.

4. Dispositif selon l'une quelconque des revendications 1 à 3, comprenant en outre un affichage.

5. Dispositif selon l'une quelconque des revendications 1 à 4, comprenant en outre un boîtier extérieur.

6. Dispositif selon l'une quelconque des revendications 1 à 5, dans lequel le système microfluidique externe (18) comprend un ou plusieurs canaux microfluidiques, une ou plusieurs connexions microfluidiques, une ou plusieurs pompes et une ou plusieurs valves pour manipuler l'échantillon, des tampons et des déchets fluides.

7. Procédé d'utilisation du dispositif selon l'une quelconque des revendications précédentes, pour la détection et la quantification d'un analyte dans un échantillon, comprenant les étapes suivantes :
a) introduction dans un récipient de l'échantillon à analyser, de billes magnétiques revêtues d'une sonde de capture et d'une sonde rapporteuse pour former une suspension comprenant des complexes sonde rapporteuse, analyste, sonde de capture et billes magnétiques,
b) introduction de la suspension résultante dans la chambre de purification (4) d'une cartouche jetable (8) à travers une entrée microfluidique (1) ;
c) purification les complexes sonde rapporteuse, analyte, sonde de capture et billes magnétiques dans la chambre de purification (4) en actionnant de façon synchronisée les deux électroaimants externes (13, 13') pour retenir, relâcher et secouer les complexes sonde rapporteuse, analyte, sonde de capture et billes magnétiques présents dans l'échantillon dans la chambre de purification (4),
d) transfert dans la chambre de mesure (5) des complexes purifiés sonde rapporteuse, analyte, sonde de capture et billes magnétiques résultants de l'étape précédente c) à travers le second canal microfluidique (11),
e) actionnement de l'aimant permanent (14) pour retenir les complexes sonde rapporteuse, analyte, sonde de capture et billes magnétiques sur la surface de l'électrode de travail de la puce (6),
f) détection et quantification de l'analyte par des moyens électrochimiques.

8. Procédé selon la revendication 7, dans lequel la purification des complexes sonde rapporteuse, analyte, sonde de capture et billes magnétiques dans la chambre de purification (4) comprend les étapes consistant à
(i) actionner les deux électroaimants externes (13, 13') pour retenir les complexes sonde rapporteuse, analyte, sonde de capture et billes magnétiques sur au moins une partie de la surface interne de la chambre de purification (4),
(ii) laisser s'écouler le reste de l'échantillon présent dans la chambre de purification (4) via le canal microfluidique (10) et une première sortie microfluidique de déchet (2),
(iii) introduire une solution tampon dans la chambre de purification (4) à travers l'entrée microfluidique (1),
(iv) éteindre les deux électroaimants externes (13, 13') pour relâcher les complexes sonde rapporteuse, analyte, sonde de capture et billes magnétiques, et les remettre en suspension dans le tampon,
(v) actionner les deux électroaimants externes (13, 13') pour agiter les complexes sonde rapporteuse, analyte, sonde de capture et billes magnétiques dans le tampon dans la chambre de purification (4),
(vi) éventuellement répéter les étapes (i) à (v).

9. Procédé selon la revendication 7 ou 8, dans lequel la détection et la quantification de l'analyte par des moyens électrochimiques sont effectuées par mesure de voltampérométrie, d'ampérométrie ou d'impédance.

10. Procédé selon la revendication 9, qui comprend :
- la connexion de la puce (6) au potentiostat (15) par des contacts électriques (7)
- le remplissage de la chambre de mesure (5) avec un tampon contenant une substance médiateur de transfert d'électrons,
- la surveillance du signal électrochimique initial, le lancement de la procédure de détection électrochimique en utilisant le potentiostat,
- l'ajout du substrat pour déclencher une réaction redox,
- l'enregistrement du décalage du signal électrochimique produit par une réaction redox,
- la traduction du décalage du signal électrochimique résultant en une valeur numérique sur l'affichage.
